# EUROPÄISCHE PATENTANMELDUNG

(11) **EP 4 660 607 A1**
(43) Veröffentlichungstag der Anmeldung: **10.12.2025**
(21) Anmeldenummer: 24179658.0
(22) Anmeldetag: 03.06.2024
(51) Int. Cl.: G01N 15/06, G01N 33/00, G01N 33/15, G01N 33/36

(54) **VERFAHREN ZUR BESTIMMUNG DES GEHALTS AN MINERALISCHER PARTIKEL IN FORMULIERUNGEN**

(71) Anmelder: Dr. Kurt Wolff GmbH & Co. KG, 33611 Bielefeld (DE)
(72) Erfinder: ENAX, Joachim, 33611 Bielefeld (DE); SCHULZE ZUR WIESCHE, Erik, 33611 Bielefeld (DE); FANDRICH, Pascal, 33611 Bielefeld (DE)
(74) Vertreter: Weickmann & Weickmann PartmbB

(57) **Zusammenfassung**

Die Erfindung betrifft ein Verfahren zur Bestimmung des Gehalts an mineralischen Partikeln in einer Formulierung und insbesondere in einer Zahnpasta.

## Beschreibung

Die Erfindung betrifft ein Verfahren zur Bestimmung des Gehalts an mineralischen Partikeln in einer Formulierung und insbesondere in einer Zahnpasta.

Mineralische Partikel sind anorganische Teilchen, die aus natürlichen oder synthetischen Quellen stammen und in verschiedenen Größen und Formen vorliegen können.

Mineralische Partikeln werden in zahlreichen Produkten eingesetzt. Hierzu zählen z. B. kosmetische Produkte, pharmazeutische Produkte, Lebensmittel, Farben und Lacke. Die mineralischen Partikel erfüllen dabei unterschiedliche Funktionen. Beispielsweise können sie wichtig sein für Funktion, Farbe, Textur, Reinigung, Schutz, Stabilisierung und Glanz der jeweiligen Produkte. Außerdem können sie als Füllstoff und Verdickungsmittel dienen. In der Regel sind mineralische Partikel sehr klein und hellgrau bis weiß, so dass sie mit dem Lichtmikroskop aufgrund des schwachen Kontrasts zumeist nicht verlässlich detektiert werden können.

Ein häufig verwendeter Mineralientyp ist Titandioxid. Titandioxid wird in zahlreichen Produkten als Weißpigment verwendet. Beispiele hierfür sind Sonnenschutzprodukte, Zahnpasten und Wandfarben. Calciumphosphate werden ebenfalls häufig eingesetzt, z.B. in Form von Hydroxylapatit in Mundpflegeprodukten zur Remineralisation der Zähne und Kariesschutz (M. Pawinska, E. Paszynska, H. Limeback, B.T. Amaechi, H.-O. Fabritius, B. Ganss, K. O'Hagan-Wong, E. Schulze zur Wiesche, F. Meyer, J. Enax, Hydroxyapatite as an active ingredient in oral care: an international symposium report, Bioinspired Biomim. Nanobiomaterials 13 (2024) 1-14).

Neben der Auswahl eines geeigneten Mineraltyps für die entsprechende Anwendung ist insbesondere auch der Gehalt der mineralischen Partikel von großer Bedeutung für die Funktion im jeweiligen Produkttyp. So ist beispielsweise bekannt, dass der Schutz vor UV-Strahlen in Sonnenschutzprodukten mit steigendem Gehalt an Titandioxid zunimmt. Ein weiteres Beispiel ist, dass eine steigende Konzentration an Silica-Putzkörpern in Zahnpasten positiv mit der Reinigungsleistung zur Entfernung von Zahnbelägen korreliert. Auch ist bekannt, dass je höher die Einsatzkonzentration von Calciumphosphaten wie Hydroxylapatit in Zahnpasten ist, desto größer deren Wirksamkeit ist.

Zur Bestimmung der Gehalte von mineralischen Partikeln in Formulierungen können unterschiedliche Methoden verwendet werden. Hierzu zählen Atomabsorptionsspektroskopie (AAS), Röntgenfluoreszenzspektroskopie (XRF), induktiv gekoppelte Plasma-Atomemissionsspektroskopie (ICP-AES), UV-VIS-Spektroskopie oder Infrarot (IR)-Spektroskopie.

Alle diese Methoden haben allerdings den Nachteil, dass sie zeitaufwendig, technisch aufwendig und kostenintensiv sind sowie eine komplexe Probenvorbereitung voraussetzen. Außerdem erfordert die Probenvorbereitung, die Durchführung der Messung und die Auswertung ein fundiertes Expertenwissen.

Die Aufgabe der Erfindung besteht somit in der Schaffung eines neuen Verfahrens zur einfachen und schnellen Bestimmung des Gehalts von mineralischen Partikeln in unterschiedlichen Formulierungen, welches die oben beschriebenen Nachteile des Standes der Technik vermeidet.

Ein erster Aspekt der Erfindung betrifft ein Verfahren zur Bestimmung des Gehalts an mineralischen Partikeln in einer Formulierung mit einer Viskosität im Bereich von 1 mPas bis 200.000 mPas,
wobei das Verfahren die Schritte umfasst
(a) (a) Auftragen der Formulierung auf eine Testmatrix, bevorzugt einen Teststreifen, wobei die Oberfläche der Testmatrix, bevorzugt des Teststreifens, auf welcher der Auftrag der Formulierung erfolgt, eine definierte Rauigkeit mit einer mittleren arithmetischen Höhe im Bereich von 0,1 bis 500 µm aufweist,
(b) Bestimmung und Auswertung des Mineraliengehalts.

Durch das erfindungsgemäße Verfahren ist es möglich den Gesamtmineralgehalt in unterschiedlichen Formulierungen zu bestimmen. Dabei kann sowohl die Messung als auch die Auswertung insbesondere auch von fachfremden Personen, insbesondere auch Endverbrauchern, schnell und zuverlässig durchgeführt werden.

Ein Anwendungsgebiet ist die Qualitätskontrolle, z. B. zur Sicherstellung des angestrebten Anteils der mineralischen Partikel in Formulierungen.

Ein weiteres Anwendungsgebiet ist die Bestimmung des Gehalts an mineralischen Partikeln in unbekannten Formulierungen.

Insbesondere wird erfindungsgemäß auch ein Verfahren bereitgestellt, welches es ermöglicht, den Gesamtmineralgehalt von mindestens zwei Formulierungen durch parallelen Auftrag auf wenigstens eine Testmatrix, bevorzugt wenigstens einen Teststreifen oder insbesondere durch Verwendung mindestens zwei Teststreifen einfach und schnell relativ zueinander vergleichen zu können.

Das erfindungsgemäße Verfahren eignet sich zur Bestimmung des Gehalts an mineralischen Partikeln in einer Formulierung mit einer Viskosität im Bereich von 1 mPas bis 200.000 mPas.

In erfindungsgemäßen Ausführungsformen betreffend Pasten und insbesondere Zahnpasten liegt die Viskosität bevorzugt im Bereich 1 - 150.000 mPas, stärker bevorzugt zwischen 5.000 -120.000 mPas und besonders bevorzugt im Bereich 10.000 - 90.000 mPas (jeweils 0-4 Tage nach Herstellung bestimmt).

In erfindungsgemäßen Ausführungsformen betreffend Lösungen und insbesondere Mundspülungen liegt die Viskosität bevorzugt im Bereich 1 - 1.000 mPas, stärker bevorzugt zwischen 1 - 500 mPas und besonders bevorzugt im Bereich 10 - 150 mPas (jeweils 0-4 Tage nach Herstellung bestimmt).

Alle Viskositätsangaben beruhen auf einer Messung nach DIN 53019-1:2008-09 mit einem Rheomter des Typs Haake RheoStress1 (ThermoFisher Scientific) bei 20 °C und einer Schwergeschwindigkeit von 10/s (Zahnpasta) und 50/s (Mundspülung) in Platte-Platte Geometrie.

Die Oberfläche der Testmatrix, bevorzugt des Teststreifens, auf welche der Auftrag der Formulierung erfolgt, weißt eine definierte Rauigkeit mit einer mittleren arithmetischen Höhe im Bereich von 0,1 bis 500 µm auf, d.h. ist rau, bzw. weist eine Körnung auf. Überraschenderweise wurde dabei gefunden, dass das Verfahren auf anorganischen Oberflächen mit einer mittleren arithmetischen Höhe der Oberfläche zwischen 0,1 µm und 500 µm besonders zuverlässig funktioniert.

Die Testmatrixoberfläche, bevorzugt Teststreifenoberfläche, kann auf einem beliebigen Trägermaterial aufgebracht sein. Das Trägermaterial kann dabei starr oder flexibel sein. Das Trägermaterial wird bevorzugt ausgewählt aus Kunstoffen, Papier, Verbundmaterialien aus Zellstoff, wie beispielsweise Vulkanfieber und/oder Leinen und Gewebe.

Eine anorganische Testmatrixoberfläche, insbesondere Teststreifenoberfläche, kann bevorzugt sein. Eine anorganische Testmatrixoberfläche, bevorzugt Teststreifenoberfläche, kann aus jedem geeignetem anorganischen Material ausgebildet sein und ist bevorzugt ausgebildet aus Siliziumkarbid, Diamant, Zirkonoxid und/oder Aluminiumoxid. Siliziumkarbid ist dabei insbesondere bevorzugt.

Die Rauheit oder Körnung der Testmatrixoberfläche, bevorzugt Testreifenoberfläche, kann entsprechend der von Schleifpapier bekannten Systematik charakterisiert werden. Bevorzugt wird eine Körnung zwischen P10 und P10000 nach FEPA (Fédération Européenne des Fabricants de Produitd Abrasifs) verwendet. Stärker bevorzugt wird eine Oberfläche mit einer Körnungsangabe zwischen P40 und P2000. Besonders bevorzugt wird eine Oberfläche mit einer Körnungsangabe zwischen P60 und P400.

In bevorzugten Ausführungsformen wird als Testmatrix, bevorzugt Teststreifen, Schleifpapier verwendet. In besonders bevorzugten Ausführungsformen kann kommerziell erhältliches Schleifpapier verwendet werden. Das verwendete Schleifpapier ist bevorzugt auf einer zusätzlichen Stabilisierungsschicht aufgebracht.

Ein Schleifpapier ist definiert als ein Werkzeug zum Abtragen von Materialien durch die Verwendung von abrasiven Körnern auf einer Trägerschicht. Die Oberfläche eines gängigen Schleifpapiers besteht aus einer Schicht aus abrasiven Körnern, die auf eine Trägerschicht aufgebracht sind. Die Körner sind in einer bestimmten Korngröße und -verteilung angeordnet, um eine gleichmäßige Schleifwirkung zu erzielen. Die Trägerschicht kann aus Papier, Gewebe oder Kunststoff bestehen und ist in der Regel flexibel, um sich den Konturen der zu schleifenden Oberfläche anzupassen. Die Körner können aus verschiedenen Materialien wie Siliziumkarbid, Diamant, Zirkonoxid oder Aluminiumoxid bestehen, je nach Anwendungsbereich und gewünschter Schleifwirkung. Die Oberfläche des Schleifpapiers kann je nach Korngröße und -art eine unterschiedliche Rauheit aufweisen.

Es können Schleifpapiere mit einer Körnung zwischen P10 und P10000 nach FEPA verwendet werden. Bevorzugt werden Schleifpapiere mit einer Körnungsangabe zwischen P40 und P2000. Besonders bevorzugt werden Schleifpapiere mit einer Körnungsangabe zwischen P60 und P400.

Für die Ausführung der erfindungsgemäßen Methode sind beispielsweise die folgenden Schleifpapiere auf Basis von Siliziumkarbid wie P80 - P3000 (Hawerk), P120 - P5000 (BBbanda) oder 120 Cw - 10000 Cw (Yuehao Abrasive Materials Co.) geeignet. Besonders bevorzugt ist Schleifpapier auf Basis von Siliziumcarbid mit der Körnungsangabe P80 (Hawerk).

Alternativ kann ein Druckverfahren verwendet werden, um eine definierte Oberflächenrauigkeit zu erzeugen. Das Druckverfahren kann ausgewählt sein aus den Verfahren Blasendruck, Hochdruck (Reliefdruck), Tiefdruck, Siebdruck, UV-Spotlackierung, Thermografie, digitaler 3D-Druck, Frostdruck oder Flockdruck.

Blasendruck: Methode bei der eine raue Struktur durch das Trocknen von Blasen in der Tinte oder einer anderen Flüssigkeit erzeugt wird.

Hochdruck (Reliefdruck): Bei dieser Methode, zu der auch der Holzschnitt und der Linolschnitt gehören, wird die Textur durch das Stehenlassen von Bereichen auf der Druckplatte erzeugt. Die erhabenen Teile werden eingefärbt und auf das Papier gedruckt, wodurch eine texturierte Oberfläche entsteht.

Tiefdruck: Beim Tiefdruck, einschließlich Radierung und Aquatinta, wird die Tinte in vertiefte Bereiche der Druckplatte eingelagert. Durch die Variation der Tiefe und der Rauheit dieser Vertiefungen können unterschiedliche Texturen im Druck erzeugt werden.

Siebdruck: Durch das Beimischen von Verdickungsmitteln oder anderen Zusätzen zur Siebdruckfarbe kann eine dreidimensionale Textur erzeugt werden. Diese Technik wird auch als Hochdruck-Siebdruck bezeichnet.

UV-Spotlackierung: Bei dieser Methode wird ein UV-reaktiver Lack auf bestimmte Bereiche des Drucks aufgetragen und dann unter UV-Licht ausgehärtet. Dies schafft eine erhabene, glänzende Oberfläche, die eine fühlbare Textur erzeugt.

Thermografie: Bei der Thermografie wird ein spezielles Pulver auf die noch feuchte Tinte gestreut und anschließend erhitzt. Das Pulver schmilzt und bläht sich auf, wodurch eine erhabene Oberfläche entsteht.

Digitaler 3D-Druck: Moderne digitale Drucker können Schichten von Tinte oder anderen Materialien auftragen, um eine texturierte Oberfläche zu schaffen.

Frostdruck: Spezielle Drucktechnik, die verwendet wird, um eine matte, raue oder strukturierte Oberfläche zu erzeugen, die an gefrorenes Glas oder Eiskristalle erinnert.

Flockdruck: Beim Flockdruck werden kurze Fasern auf den Druck aufgebracht, während die Tinte noch feucht ist. Die Fasern haften an der Tinte und erzeugen eine samtige, texturierte Oberfläche.

Die Testmatrix und insbesondere der Teststreifen, ist bevorzugt dadurch charakterisiert, dass die mittlere arithmetische Höhe der Oberfläche gemessen mittels Laserrastermikroskopie im Bereich von 0,1 µm bis 500 µm, insbesondere 1,0 µm bis 300 µm, liegt. Bevorzugt liegt die mittlere arithmetische Höhe der Oberfläche der Testmatrix, bevorzugt des Teststreifens, im Bereich 5 µm bis 250 µm, stärker bevorzugt 10 µm bis 100 µm, noch stärker bevorzugt im Bereich 40 µm bis 60 µm. Die Messung der mittleren arithmetischen Höhe der Oberfläche der Testmatrix, bevorzugt des Teststreifens, kann beispielsweise mit einem Keyence Laserrastermikroskop VK-X 1100 erfolgen.

Die Oberfläche der Testmatrix, bevorzugt des Testreifens, ist bevorzugt in einer Farbe gehalten, die einen guten Kontrast zu den zu analysierenden mineralischen Partikeln, in der Regel hellgrau bis weiß, bietet. Bevorzugt sind hier dunkle und/oder gedeckte Farben und insbesondere schwarz, blau, grün oder rot.

Die erfindungsgemäße Testmatrix, bevorzugt Testreifen, kann jede für den jeweiligen Zweck geeignete Abmessungen aufweisen. Eine typische Abmessung ist beispielsweise 2x10 cm. Die Testmatrix kann erfindungsgemäß aber auch quadratisch sein.

Das Auftragen der Formulierung auf die Testmatrix, bevorzugt den Teststreifen, gemäß Schritt (A) erfolgt bevorzugt homogen.

Die Formulierung, die die mineralischen Partikel enthält, kann auf jede geeignete Weise auf die Testmatrix, bevorzugt den Testreifen, aufgebracht werden. Bevorzugt erfolgt der Auftrag auf die Testmatrix, bevorzugt den Teststreifen, mithilfe des Fingers, einer Zahnbürste, einem Spatel, einem Spachtel, einem Pinsel, einer Rolle oder einem Schwamm aufgetragen werden. Besonders bevorzugt ist die Auftragung mit einer Zahnbürste oder einem Spatel.

Nach dem Auftrag können die flüchtigen Bestandteile wie Wasser oder organische Lösemittel durch Trocknen in einem Trocknungsschritt (a') entfernt werden. Diese Trocknung kann bevorzugt an der Luft bei Raumtemperatur erfolgen oder stärker bevorzugt durch Erhitzen, beispielsweise mit einem Föhn oder in einem Wärmeschrank.

Erfindungsgemäß können mehrere zu analysierende Formulierungen nebeneinander aufgetragen und somit gleichzeitig analysiert werden.

Die Auswertung der Testmatrix, bevorzugt des Teststreifens, kann visuell oder digital erfolgen.

Eine bevorzugte Auswertung erfolgt visuell über den Bedeckungsgrad der Testmatrix, bevorzugt des Teststreifens.

Überraschenderweise wurde festgestellt, dass der Mineralgehalt der Formulierung nach dem Auftrag auf dem Teststreifen ohne sichtbare Bedeckung des Teststreifens kleiner 1 Gew.-% beträgt, bei einer mäßigen Bedeckung des Teststreifens im Bereich 1 bis 5 Gew.-% beträgt, bei einer überwiegenden Bedeckung des Teststreifens im Bereich 5 bis 10 Gew.-% beträgt und bei einer vollständigen Bedeckung des Teststreifens größer 10 Gew.-% beträgt.

Eine Auswertung des Mineraliengehalts kann nach dem Auftrag durch den Vergleich des Grautons des Auftrags auch mit entsprechenden Referenzwerten oder einer Referenzskala erfolgen. Die Auswertung kann beispielsweise mithilfe einer Grautonskala erfolgen, z. B. gedruckt auf Papier, z. B. Sigel Laser-Papier, LP207, DIN A4, weiß gedruckt mit einem Drucker von Konica Minolta, Typ bizhub C450i (Abbildung A).

Der Mineralgehalt der zu analysierenden Formulierung kann auch visuell relativ zu einer Referenzformulierung bestimmt werden. Es ist erfindungsgemäß auch möglich, den Mineralgehalt zwischen mindestens zwei zu analysierenden Formulierungen relativ zueinander zu bestimmen, vorzugsweise visuell.

Neben der visuellen Auswertung kann alternativ eine digitale Auswertung von Grauwerten mittels aufgenommen Fotos erfolgen. Die Grauwerte können beispielsweise mit Softwareprodukten wie ImageJ (National Insitutes of Health, Bethesda, USA) oder Adobe Photoshop^{®} (Adobe Inc.) bestimmt werden. Für den Vergleich zwischen einzelnen Fotos können Referenzwerte aus der Grautonskala ermittelt und herangezogen werden.

Das erfindungsgemäße Verfahren ermöglicht die Bestimmung des Gehalts an mineralischen Partikeln in Formulierungen insbesondere ausgewählt aus kosmetischen Produkten, pharmazeutischen Produkten, Lebensmitteln, Farben und/oder Lacken.

Mit dem erfindungsgemäßen Verfahren kann der Gehalt von anorganischen Substanzen bestimmt werden. So kann beispielsweise insbesondere der Gehalt von Calciumphosphaten, Titandioxid, Zinkoxid, Talkum, Kaolin, Mica, Eisenoxiden, Ultramarin, Zinnoxid, Zirkoniumdioxid, Silikaten, Mischsilikaten, Schichtsilikaten, Carbonaten, Aluminiumoxiden, Aluminiumsilikaten, Bariumsulfat, Chromoxidgrün, Cadmiumsulfid, Zinnober, Manganviolett, Chromoxidbraun, Bismutoxid oder Kobaltblau in unterschiedlichen Formulierungen bestimmt werden.

Verfahrensgemäß können Calciumphosphate aus der Gruppe bestehend aus Monocalciumphosphat-Monohydrat (MCPM), Monocalciumphosphat-Anhydrat (MCPA), Dicalciumphosphat-Dihydrat (DCPD, Brushit), Dicalciumphosphat-Anhydrat (DCPA, Monetit), Octacalciumphosphat (OCP), α-Tricalciumphosphat (α-TCP), β-Tricalciumphosphat (β-TCP), amorphem Calciumphosphat (ACP; auch als CPP-ACP-Komplex = Casein Phosphopeptid - amorphes Calciumphosphat), amorphem Calciumphosphat (ACP), Calcium-defizitärem Hydroxylapatit (CDHA), Hydroxylapatit (HA oder HAP), Tetracalciumphosphat (TTCP) und Calciumpyrophosphat, bevorzugt aus der Gruppe bestehend aus Monocalciumphosphat-Monohydrat (MCPM), Monocalciumphosphat-Anhydrat (MCPA), Dicalciumphosphat-Dihydrat (DCPD, Brushit), Dicalciumphosphat-Anhydrat (DCPA, Monetit), Octacalciumphosphat (OCP), amorphem Calciumphosphat (ACP; auch als CPP-ACP-Komplex = Casein Phosphopeptid - amorphes Calciumphosphat), amorphem Calciumphosphat (ACP), Calcium-defizitärem Hydroxylapatit (CDHA), Hydroxylapatit (HA oder HAP) und Tetracalciumphosphat (TTCP) und jegliche Mischungen dieser Verbindungen untersucht werden, bevorzugt Hydroxylapatit.

Bevorzugt ist die Bestimmung des Gehalts an mineralischen Partikeln in Mundpflegeprodukten ausgewählt aus Zahnpasten, Mundspülungen, Oralgelen oder Polierpasten.

Bevorzugt ist die Bestimmung des Gesamtmineralgehalts von Zahnpasten und insbesondere die Bestimmung von Hydroxylapatit in Zahnpasten. Besonders bevorzugt ist der relative Vergleich der Hydroxylapatit-Gehalte von mindestens zwei Zahnpasten.

Eine Zahnpasta bzw. eine Zahncreme ist eine spezielle Paste bzw. Creme, die zur Reinigung der Zähne und zur Vorbeugung von Karies und Zahnfleischerkrankungen verwendet wird.

Ein Oralgel ist eine spezielle Gel-Substanz, die zur Behandlung von Zahnfleischentzündungen, Mundgeschwüren oder anderen oralen Beschwerden sowie zur Vorbeugung von Karies und anderen Zahnerkrankungen verwendet wird.

Eine Mundspülung ist eine Flüssigkeit, die zur Reinigung des Mundraums, zur Bekämpfung von Mundgeruch und zur Vorbeugung von Karies und Zahnfleischerkrankungen verwendet wird.

Eine Polierpaste ist eine spezielle Paste, die zur Glättung und Politur von Zähnen und Zahnersatz verwendet wird, um eine glatte und ästhetisch ansprechende Oberfläche zu erzielen.

Ebenfalls bevorzugt ist die Bestimmung von mineralischen Partikeln in Sonnenschutzprodukten ausgewählt aus Sonnencremes, Sonnenmilch, Sonnensprays, Sonnenölen oder Lippenpflegeprodukten. Besonders bevorzugt ist die Bestimmung von Titandioxid in Sonnencremes und Sonnenmilch.

Sonnencremes sind spezielle Cremes, die auf die Haut aufgetragen werden, um vor Sonnenbrand und UV-Strahlung zu schützen.

Sonnenmilch ist eine flüssige Sonnenschutzlotion, die auf die Haut aufgetragen wird, um vor Sonnenbrand und UV-Strahlung zu schützen.

Sonnensprays sind Sprühprodukte, die auf die Haut aufgetragen werden, um vor Sonnenbrand und UV-Strahlung zu schützen.

Sonnenöle sind Öle, die auf die Haut aufgetragen werden, um vor Sonnenbrand und UV-Strahlung zu schützen.

Lippenpflegeprodukte sind spezielle Produkte, die auf die Lippen aufgetragen werden, um sie vor Austrocknung, Sonnenbrand und UV-Strahlung zu schützen.

Ebenfalls bevorzugt ist die Bestimmung von mineralischen Partikeln in Wandfarben.

Eine Wandfarbe ist eine spezielle Farbe, die zum Anstrich von Innen- und Außenwänden verwendet wird, um sie zu schützen und zu dekorieren.

Eine erfindungsgemäß besonders bevorzugte Ausführungsform betrifft ein Verfahren zur Bestimmung des Gehalts an Hydroxylapatit in einer Zahnpasta
wobei das Verfahren die Schritte umfasst
(a) Auftragen der Zahnpasta auf eine erfindungsgemäße Testmatrix, bevorzugt Teststreifen,
(a') Trocknen der aufgetragenen Zahnpasta und
(b) Bestimmung des Gehalts von Hydroxylapatit durch Vergleich mit Referenzwerten, insbesondere anderen Zahnpasten.

Ein Aspekt der Erfindung betrifft einen Kit umfassend eine erfindungsgemäße Testmatrix, bevorzugt wenigstens einen erfindungsgemäßen Teststreifen, eine Anleitung zur Durchführung des erfindungsgemäßen Verfahrens, und eine Formulierung, die nach dem erfindungsgemäßen Verfahren analysiert werden kann. Hierbei ist ein Kit bevorzugt, der wenigstens einen erfindungsgemäßen Teststreifen, eine Anleitung zur Durchführung des erfindungsgemäßen Verfahrens und ein Zahnpflegeprodukt, insbesondere Zahnpasta, umfasst. Der Kit ermöglicht dem Verbraucher hinsichtlich des Mineraliengehalts und insbesondere des Gehalts an Hydroxylapatit einen eigenständigen Vergleich der im Kit enthaltenen Zahnpasta mit anderen Zahnpasten.

### Abbildungen:

- **Abbildung A:**: Beispielhafte Grautonskala gedruckt auf Papier, hier Sigel Laser-Papier, LP207, DIN A4, weiß gedruckt mit einem Drucker von Konica Minolta, Typ bizhub C450i.
- **Abbildung B1-1:**: Die Teststreifen zeigen unterschiedliche Bedeckungen, in Abhängigkeit der verwendeten Zahnpasta.
- **Abbildung B1-2:**: Die ermittelten mittleren Grauwerte der Teststreifen aufgetragen gegen den Hydroxylapatitgehalt.
- **Abbildung B2-1:**: Einteilung der Grautontabelle in unterschiedliche Bewertungsbereiche.
- **Abbildung B3-1:**: Oben: Die erfindungsgemäßen Teststreifen mit anorganischer Oberfläche zeigen eine homogene Bedeckung der Zahnpastaformulierungen. Unten: Die Verwendung von cellulosehaltigem Karton als Testuntergrund führt zu inhomogener Bedeckung.
- **Abbildung B4-1:**: Die Teststreifen zeigen unterschiedliche Bedeckungen in Abhängigkeit der verwendeten Sonnencreme.
- **Abbildung B4-2:**: Die ermittelten mittleren Grauwerte der Teststreifen aufgetragen gegen den Titandioxidgehalt.

### Beispiele

Nachstehend wird die Erfindung anhand von Beispielen erläutert.

### Beispiel 1: Bestimmung des Mineralgehalts in Zahnpasten durch den erfindungsgemäßen Teststreifen

Für die Bestimmung des Gesamtmineralgehalts in Zahnpasten werden die folgenden Formulierungen verwendet:

| | Bezeichnung | | | | | | | | |
|---|---|---|---|---|---|---|---|---|---|
| Inhaltsstoffe (Anteile in Gew.-%) | A | B | C | D | E | F | Z1 | Z2 | Z3 |
| **Hydroxylapatit** | 0,0 | 1,0 | 5,0 | 10,0 | 20,0 | 36,7 | 0,0 | 4,0 | 22,0 |
| **hydratisierte Kieselsäure** | - | - | - | - | - | - | 3,0 | 3,0 | 3,0 |
| Glycerin | 10,0 | | | | | | | | |
| Sorbitol | 4,0 | | | | | | | | |
| Xylitol | 2,0 | | | | | | | | |
| Zellulosegummi | 1,0 | | | | | | | | |
| Hydroxyethylcellulose | 0,2 | | | | | | | | |
| Carboxymethylcellulose | 0,2 | | | | | | | | |
| Natriummethylcocoyltaurat | 2,0 | | | | | | | | |
| Natriumchlorid | 0,7 | | | | | | | | |
| Benzylalkohol | 0,5 | | | | | | | | |
| Phenoxyethanol | 0,4 | | | | | | | | |
| Natriumbenzoat | 1,0 | | | | | | | | |
| Natriumlaurylsulfat | 0,1 | | | | | | | | |
| Natriummyristoylsarkosinat | 1,5 | | | | | | | | |
| Natriumsaccharin | 0,5 | | | | | | | | |
| Zinkchlorid | 0,1 | | | | | | | | |
| Carrageenan | 3,0 | | | | | | | | |
| Xanthangummi | 0,2 | | | | | | | | |
| | Rest: demineralisiertes Wasser | | | | | | | | |
| **Gesamtmineralgehalt** (Hydroxylapatit + hydratisierte Kieselsäure) | **0,0** | **1,0** | **5,0** | **10,0** | **20,0** | **36,7** | **3,0** | **7,0** | **25,0** |

Es werden die Zahnpasten A bis F mit den oben genannten Mengen Hydroxylapatit (HAP) hergestellt.

0,3 g der Zahnpasten A bis F werden jeweils, entsprechend Abbildung B1-1, homogen auf den Teststreifen verteilt. Bei der Verteilung wird die Zahnpasta auf dem Teststreifen platziert und in Richtung des Streifenendes ausgestrichen.

Die Oberfläche der Teststreifen besteht aus Siliziumcarbid mit einer mittleren Korngröße von 201 µm. Die bedeckten Teststreifen werden mit einem Föhn (AD31, Anruzon) vollständig getrocknet und gemeinsam fotografiert. Gemäß Abbildung B1-1 zeigen die Teststreifen, abhängig vom Hydroxylapatitgehalt, unterschiedliche Bedeckungen. Die Bedeckung reicht von keiner, bis zu einer vollständigen Bedeckung.

Das erzeugte Bild mit den auszuwertenden Teststreifen wird mit einem Bildbearbeitungsprogramm (ImageJ, National Insitutes of Health, Bethesda, USA) in ein 8-Bit Graustufenbild überführt. Mit Hilfe der Software werden die mittleren Grauwerte im Bedeckungsbereich der Teststreifen bestimmt.

Die mittleren Grauwerte werden gegen den Hydroxylapatitgehalt, gemäß Abbildung B1-2, aufgetragen.

Zur Verifizierung des Gesamtmineralgehalts einer Testzahncreme werden, die in der oben genannten Tabelle aufgeführten Zahncremes Z1 bis Z3 verwendet. Die Testzahncremes enthalten zusätzlich hydratisierte Kieselsäure, ein gängiger, mineralischer Putzkörper in Zahnpasten.

Die Teststreifen mit den Zahnpasten Z1 bis Z3 mit Gesamtmineralgehalten von 3 Gew.-%, 7 Gew.-% und 25 Gew.-% werden wie oben beschrieben vorbereitet und ausgewertet. Die Grauwerte der Testformulierungen aus einer Fünffachbestimmung sind in der folgenden Tabelle aufgeführt:

| Zahnpasta | Gesamtmineralgehalt in Gew.-% | Erwarteter Grauwertbereich It. Abb. B1-2 | Ermittelter Grauwert |
|---|---|---|---|
| Z1 | 3,0 | 86 - 144 | 130 ± 9 |
| Z2 | 7,0 | 144 - 192 | 173 ± 12 |
| Z3 | 25,0 | 192 - 213 | 209 ± 15 |

Die Grauwerte stimmen mit dem Referenzverlauf aus Abb. B1-2 überein.

Das Beispiel zeigt wie die erfindungsgemäße Methode eine Bestimmung des Gesamtmineralgehalts in Zahnpasten ermöglicht.

### Beispiel 2: Schnelltest zur Bestimmung des Mineralgehalts in Zahnpasten anhand eines Bewertungssystems

Zur Verwendung der erfindungsgemäßen Methode als Schnelltest, wird der Gesamtmineralgehalt der Zahnpasten Z1 bis Z3 aus Beispiel 1 anhand einer Bewertungsskala bestimmt. Die Teststreifen mit den Zahnpasten Z1 bis Z3 aus Beispiel 1 werden mit Hilfe einer Grautonskala optisch bewertet. Die Grautonskala wird auf weißes Papier (Sigel Laser-Papier, LP207, DIN A4, weiß) mit einem Drucker (Konica Minolta, bizhub C450i) gedruckt.

Die RGB-Werte beziehen sich auf die numerische Darstellung der Intensitäten der Grundfarben Rot, Grün und Blau. Der Wert "0" in Abbildung B2-1 repräsentiert den RGB-Wert (0, 0, 0) und somit die Farbe Schwarz. Der Wert "255" in Abbildung B2-1 repräsentiert den RGB-Wert (255, 255, 255) und somit die Farbe Weiß, beziehungsweise den Untergrund der Grautonskala. Alle Zwischenstufen entsprechen einer gleichmäßigen Kombination der Grundfarben ("100" = (100/100/100)) und somit einer Grautonabstufung.

Für die Grautonskala wird, gemäß Abbildung B2-1, ein Bewertungsschema mit den RGB-Bereichen 0 bis 100 (0 - 1 Gew.-% Gesamtmineralgehalt, kaum Bedeckung), 100 bis 150 (1 - 5 Gew.-%, mäßige Bedeckung), 150 bis 220 (5 - 10 Gew.-%, überwiegende Bedeckung) und 220 bis 255 (> 10 Gew.-%, vollständige Bedeckung) festgelegt.

Die Teststreifen Z1 bis Z3 werden in einer Fünffachbestimmung mit Hilfe der Grautonskala optisch bewertet:

| Zahnpasta | Gesamtmineralgehalt Testzahnpasta / Gew.-% | RGB-Zuordnung nach Grautonskala | Gesamtmineralgehalt gemäß Scoring-Bewertung aus Abb. B2-1 |
|---|---|---|---|
| Z1 | 3 | 140 ± 10 | 1 - 5 Gew.-% |
| Z2 | 7 | 190 ± 10 | 5 - 10 Gew.-% |
| Z3 | 25 | 245 ± 5 | >10 Gew.-% |

Das Beispiel zeigt, dass sich die erfindungsgemäße Methode in Kombination mit der angeführten Grautonskala zur schnellen und praktikablen Einordnung des Gesamtmineralgehalts in Zahnpasten eignet.

### Beispiel 3: Vergleich unterschiedlicher Testoberflächen zur Bestimmung des Gesamtmineralgehalts

Im folgenden Beispiel werden die oben beschriebenen Teststreifen mit schwarzem, auf Cellulose basiertem Karton verglichen, um die Eignung einer anderen Oberfläche zu testen. Die Teststreifen aus Beispiel 1 und 2 werden mit Teststreifen aus schwarzem Karton (2 x 10 cm, schwarz, 300 g/m², idee. Creativmarkt GmbH & Co. KG) verglichen. Hierzu werden die Kartonstreifen analog zu Beispiel 1 mit den entsprechenden Zahnpastaformulierungen präpariert.

Überraschenderweise zeigt sich bei der Verwendung schwarzen Kartons, wie in Abbildung B3-1 zu sehen, eine inhomogene, nichtauswertbare Bedeckung auf einer cellulosehaltigen Oberfläche.

Das Beispiel zeigt, dass ein homogener Auftrag nur mit den charakterisierenden Merkmalen der erfindungsgemäßen Teststreifen möglich ist.

### Beispiel 4: Bestimmung der Titandioxid-Konzentration aus Sonnencreme

Für die Bestimmung des Titandioxidgehalts in Sonnencreme werden die folgenden Formulierungen verwendet:

| Inhaltsstoffe (Anteile in Gew.-%) | G | H | I | J | K | L | S1 | S2 | S3 |
|---|---|---|---|---|---|---|---|---|---|
| **Titandioxid** | 0,0 | 1,0 | 5,0 | 10,0 | 20,0 | 36,7 | 2,0 | 7,0 | 15,0 |
| Octocrilen | 8,5 | | | | | | | | |
| Glycerin | 4,2 | | | | | | | | |
| Avobenzon | 3,0 | | | | | | | | |
| Xanthangummi | 1,3 | | | | | | | | |
| Homosalat | | | | | | | 2,5 | | |
| Octylsalicylat | | | | | | | 0,8 | | |
| Dimeticon | | | | | | | 0,01 | | |
| Vitamin E-Acetat | | | | | | | 0,001 | | |
| | Rest: demineralisiertes Wasser | | | | | | | | |
| **Gesamtmineralg ehalt** (Titandioxid) | **0,0** | **1,0** | **5,0** | **10,0** | **20,0** | **36,7** | **2,0** | **7,0** | **15,0** |

Es werden die Sonnencremes G bis L mit den oben genannten Mengen Titandioxid (TiO₂) hergestellt.

0,3 g der Sonnencremes G bis L werden jeweils, entsprechend Abbildung B4-1, homogen auf den Teststreifen verteilt. Bei der Verteilung wird die Sonnencreme auf dem Teststreifen platziert und in Richtung des Streifenendes ausgestrichen.

Die Oberfläche der Teststreifen entspricht der Oberfläche aus Beispiel 1. Die bedeckten Teststreifen werden analog zu Beispiel 1 getrocknet und gemeinsam fotografiert. Abbildung B4-1 zeigt unterschiedliche Bedeckungen, in Abhängigkeit des Titandioxidgehalts. Die Bedeckung reicht von keiner, bis zu einer vollständigen Bedeckung.

Das erzeugte Bild mit den auszuwertenden Teststreifen wird mit ImageJ in ein 8-Bit Graustufenbild überführt. Mit Hilfe der Software werden die mittleren Grauwerte im Bedeckungsbereich der Teststreifen bestimmt.

Die mittleren Grauwerte aus einer Fünffachbestimmung werden gegen den Titandioxidgehalt, gemäß Abbildung B4-2, aufgetragen.

Zur Verifizierung des Titandioxidgehalts einer Sonnencreme werden die in der oben genannten Tabelle aufgeführten Sonnencremes S1 bis S3 verwendet.

Die Teststreifen mit den Sonnencremes S1 bis S3 mit Gesamtmineralgehalten von 2 Gew.-%, 7 Gew.-% und 15 Gew.-% werden wie oben beschrieben auf den Teststreifen ausgestrichen und ausgewertet. Die Grauwerte aus einer Fünffachbestimmung der Testformulierungen sind in der folgenden Tabelle aufgeführt:

| Zahnpasta | Gesamtmineralgehalt in Gew.-% | Erwarteter Grauwertbereich It. Abb. B1-2 | Ermittelter Grauwert |
|---|---|---|---|
| S1 | 2,0 | 108 - 181 | 169 ± 14 |
| S2 | 7,0 | 181 - 209 | 193 ± 8 |
| S3 | 15,0 | 209 - 220 | 214 ± 9 |

Die Grauwerte stimmen mit dem Referenzverlauf aus Abb. B1-2 überein.

Das Beispiel zeigt, wie die erfindungsgemäße Methode eine Bestimmung des Titandioxidgehalts in Sonnencreme ermöglicht.

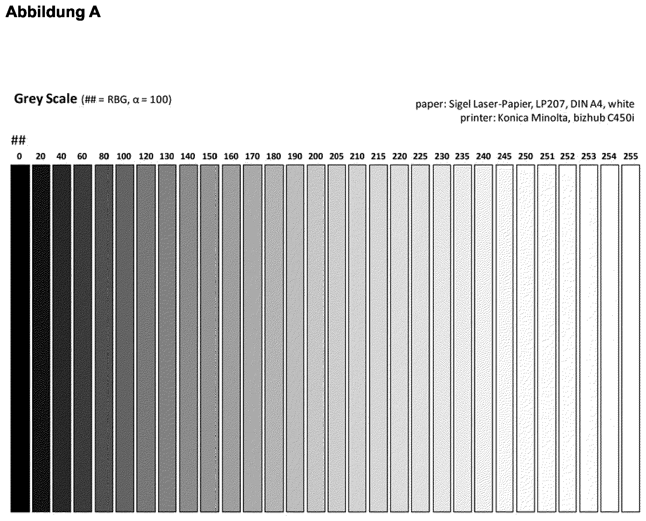

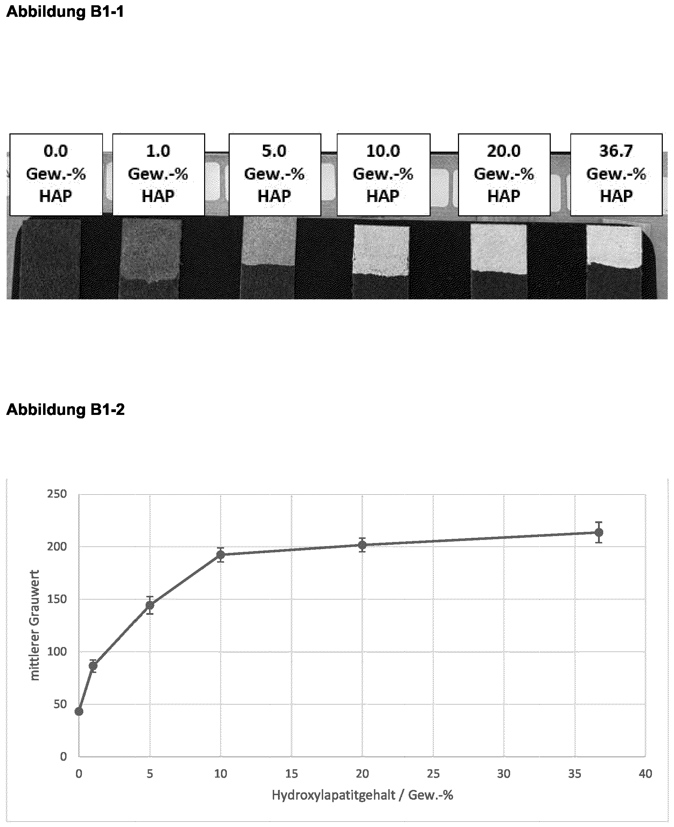

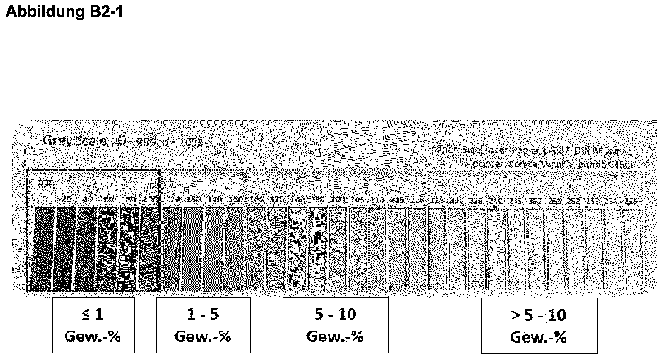

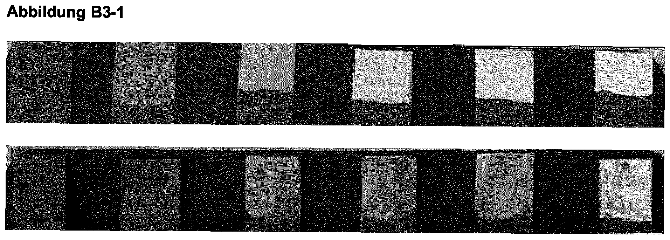

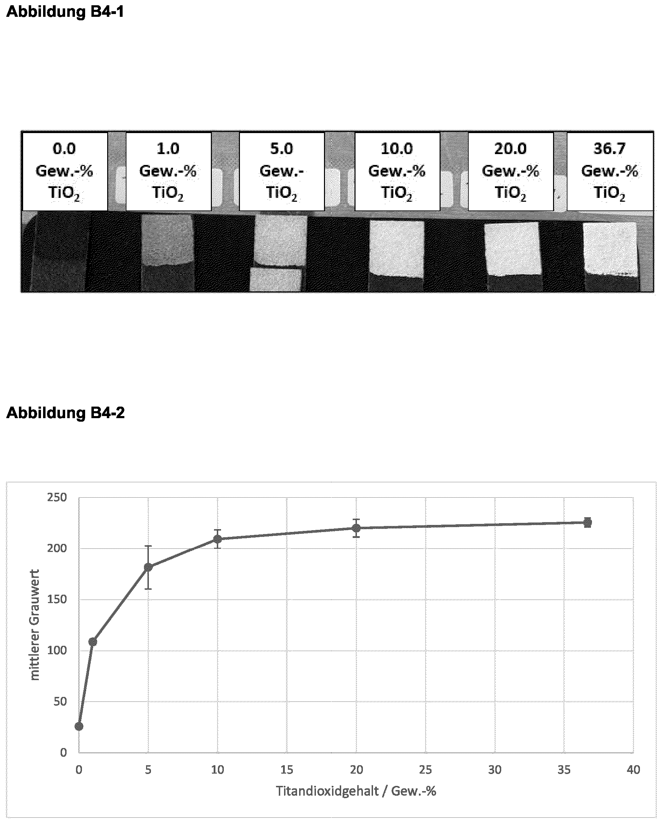

## Patentansprüche

**1.** Verfahren zur Bestimmung des Gehalts an mineralischen Partikeln in einer Formulierung mit einer Viskosität im Bereich von 1 mPas bis 200.000 mPas,
wobei das Verfahren die Schritte umfasst
(a) Auftragen der Formulierung auf eine Testmatrix, bevorzugt einen Teststreifen, wobei die Oberfläche des Testreifens auf welcher der Auftrag der Formulierung erfolgt, eine definierte Rauigkeit mit einer mittleren arithmetischen Höhe im Bereich von 0,1 bis 500 µm aufweist,
(b) Bestimmung und Auswertung des Mineraliengehalts.

**2.** Verfahren nach Anspruch 1, wobei die Oberfläche der Testmatrix auf welcher der Auftrag der Formulierung aus anorganischem Material ausgebildet ist und/oder mittels Druckverfahren aufgebracht ist.

**3.** Verfahren nach einem der vorangegangenen Ansprüche, wobei die Testmatrix abrasive Körner aus Siliziumkarbid, Diamant, Zirkonoxid oder Aluminiumoxid auf einer Trägerschicht umfasst.

**4.** Verfahren nach Anspruch 2, wobei die Druckverfahren ausgewählt ist aus Blasendruck, Hochdruck (Reliefdruck), Tiefdruck, Siebdruck, UV-Spotlackierung, Thermografie, digitaler 3D-Druck, Frostdruck oder Flockdruck.

**5.** Verfahren nach einem der vorangegangenen Ansprüche, wobei die mittlere arithmetische Höhe der Oberfläche der Testmatrix eine definierte Rauigkeit mit einer mittleren arithmetischen Höhe im Bereich von 1 bis 300 µm aufweist.

**6.** Verfahren nach einem der vorangegangenen Ansprüche, wobei die Testmatrix aus einem Schleifpapier ausgebildet ist.

**7.** Verfahren gemäß einem der vorangegangenen Ansprüche, wobei die Formulierung, in Schritt (a) mithilfe des Fingers, einer Zahnbürste, einem Spatel, einem Spachtel, einem Pinsel, einer Rolle oder einem Schwamm auf die Testmatrix aufgetragen wird.

**8.** Verfahren gemäß einem der vorangegangenen Ansprüche, wobei nach dem Auftragen der Formulierung auf die Testmatrix gemäß Schritt (a) ein Trocknungsschritt (a') erfolgt.

**9.** Verfahren gemäß einem der vorangegangenen Ansprüche, wobei mehrere zu analysierende Formulierungen parallel auf die Testmatrix aufgetragen werden.

**10.** Verfahren gemäß einem der vorangegangenen Ansprüche, wobei der Mineralgehalt einer Formulierung visuell relativ zu einer Referenzformulierung bestimmt wird.

**11.** Verfahren gemäß einem der vorangegangenen Ansprüche, wobei der Mineralgehalt von mindestens zwei Formulierungen relativ zueinander, vorzugsweise visuell, bestimmt wird.

**12.** Verfahren gemäß einem der vorangegangenen Ansprüche, wobei der Mineralgehalt einer Formulierung nach dem Auftrag auf die Testmatrix ohne sichtbare Bedeckung der Testmatrix kleiner 1 Gew.-% beträgt, bei einer mäßigen Bedeckung der Testmatrix 1 bis 5 Gew.-% beträgt, bei einer überwiegenden Bedeckung der Testmatrix 5 bis 10 Gew.-% beträgt und bei einer vollständigen Bedeckung der Testmatrix größer 10 Gew.-% beträgt.

**13.** Verfahren gemäß einem der vorangegangenen Ansprüche, wobei die Auswertung des Gehalts an mineralischer Partikel nach dem Auftrag der Formulierung durch unter Anwendung einer Referenzskala, bevorzugt unter Bestimmung der Grauwerte, erfolgt.

**14.** Verfahren gemäß einem der vorangegangenen Ansprüche, wobei der Gehalt von Mineralien ausgewählt aus Calciumphosphaten, Titandioxid, Zinkoxid, Talkum, Kaolin,
Mica, Eisenoxiden, Ultramarin, Zinnoxid, Zirkoniumdioxid, Silikaten, Mischsilikaten, Schichtsilikaten, Carbonaten, Aluminiumoxiden, Aluminiumsilikaten, Bariumsulfat, Chromoxidgrün, Cadmiumsulfid, Zinnober, Manganviolett, Chromoxidbraun, Bismutoxid und/oder Kobaltblau bestimmt wird.

**15.** Verfahren gemäß einem der vorangegangenen Ansprüche, wobei der Gehalt von mineralischen Partikeln in kosmetischen Produkten, pharmazeutischen Produkten, Lebensmitteln, Farben oder Lacken bestimmt wird.

**17.** Verfahren gemäß einem der vorangegangenen Ansprüche, wobei der Gehalt von mineralischen Partikeln in Mundpflegeprodukten bestimmt wird.

**18.** Verfahren gemäß einem der vorangegangenen Ansprüche, wobei der Gehalt von Hydroxylapatit in Zahnpasten bestimmt wird.

**19.** Verfahren gemäß einem der vorangegangenen Ansprüche, wobei der Gehalt von mineralischen Partikeln in Sonnenschutzprodukten bestimmt wird.

**20.** Verfahren gemäß einem der vorangegangenen Ansprüche, wobei der Gehalt von mineralischen Partikeln in Wandfarben bestimmt wird.

**21.** Kit umfassend eine Anleitung zur Durchführung des Verfahrens nach einem der Ansprüche 1 bis 18, eine Testmatrix, bevorzugt wenigstens einen Teststreifen, und ein Mundpflegeprodukt, bevorzugt eine Zahnpasta.
